(19) Europäisches Patentamt
European Patent Office
Office européen des brevets

(11) EP 4 659 658 A1

(12) EUROPEAN PATENT APPLICATION

(43) Date of publication:
**10.12.2025 Bulletin 2025/50**

(21) Application number: **25179660.3**

(22) Date of filing: **29.05.2025**

(51) International Patent Classification (IPC):
***A61B 5/00*** *(2006.01)* ***B32B 27/08*** *(2006.01)*

(52) Cooperative Patent Classification (CPC):
**A61B 5/1473;** A61B 5/686; A61B 2562/0215; G01N 27/301

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **31.05.2024 CN 202410704426**

(71) Applicant: **Shanghai United Imaging Microelectronics Technology Co., Ltd.**
**Shanghai 201800 (CN)**

(72) Inventor: **FANG, Wen**
**Shanghai, 201800 (CN)**

(74) Representative: **Murgitroyd & Company**
**165-169 Scotland Street**
**Glasgow G5 8PL (GB)**

(54) **SENSOR PROTECTIVE FILM AND METHOD OF PREPARATION AND USE**

(57) A sensor protective film, the film comprising: a first polymer layer and at least one additional layer, the first polymer layer and the at least one additional layer are arranged in a stacked configuration; (a) the first polymer layer comprising at least one cationic functional group selected from the group comprising: (i) a nitrogen-containing cationic functional group, or (ii) a phosphorus-containing cationic functional group; and (b) the additional layer selected from at least one of a second polymer layer, or a third polymer layer, (i) the second polymer layer comprising at least one polymer selected from the group comprising: a sulfonic acid group-containing polymer, or a silver-ion-coordinating neutral ligand-containing polymer, and (ii) the third polymer layer comprising at least one of an anionic group-containing polymer or a cationic group-containing polymer.

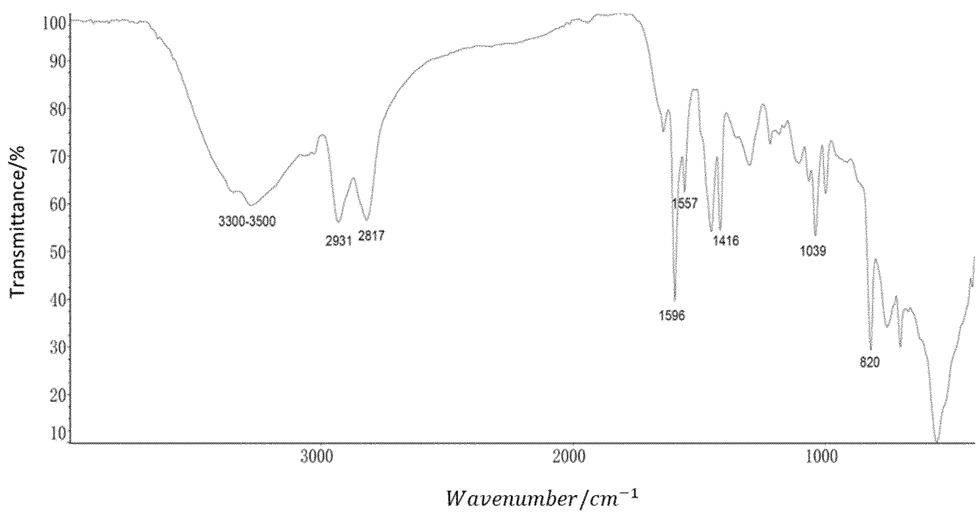


FIG. 1

EP 4 659 658 A1

## Description

### CROSS-REFERENCE TO RELATED APPLICATION

This application claims priority to Chinese Patent Application No. 202410704426.9, filed on May 31, 2024.

### TECHNICAL FIELD

The present application relates to sensor, and in particular, to sensor protective film and method of preparation and use.

### BACKGROUND

A reference electrode is an essential component of an electrochemical sensor, which is typically constructed from an Ag/AgCl material. A core requirement for the reference electrode is maintenance of stable potential; however, compositional or structural changes may cause potential drift. Due to the dissolution equilibrium: $AgCl(s) \rightleftharpoons Ag^+(aq) + Cl^-(aq)$, trace amounts of free silver ions exist within the AgCl layer. Driven by concentration gradients, these $Ag^+$ ions continuously diffuse into the external immersion medium. Leakage of $Ag^+$ from the AgCl layer, however, diminishes the reference electrode's stability, shortens the sensor's service life, and when the electrochemical sensor is implanted in a human body presents biosafety risks due to the cytotoxicity of leaked silver ions. Conventional protective membranes used in sensors have failed to effectively prevent $Ag^+$ leakage from the AgCl layer, thereby leading to compromised reference electrode stability, reduced sensor longevity, and inadequate biosafety.

Therefore, it is desirable to provide a sensor protective film and method of preparation and use.

### SUMMARY

An aspect of the present application may provide a sensor protective film, comprising: a first polymer layer and at least one additional layer, the first polymer layer and the at least one additional layer are arranged in a stacked configuration ;(a) the first polymer layer comprising at least one cationic functional group selected from the group comprising: (i) a nitrogen-containing cationic functional group, or (ii) a phosphorus-containing cationic functional group; and (b) the additional layer selected from at least one of a second polymer layer, or a third polymer layer: (i) the second polymer layer comprising at least one polymer selected from the group comprising: a sulfonic acid group-containing polymer, or a silver-ion-coordinating neutral ligand-containing polymer, (ii) the third polymer layer comprising at least one of an anionic functional group or a cationic functional group.

In some embodiments, a sensor protective film comprises a first polymer layer, a second polymer layer, and a third polymer layer sequentially arranged stacked.

In some embodiments, a second polymer layer is covalently bonded to each of a first polymer layer and a third polymer layer.

In some embodiments, the cationic functional group in the first polymer layer is selected from at least one of a positively charged nitrogen heterocyclic structure, a quaternary ammonium cation, or a quaternary phosphine cation; the cationic functional group in the first polymer layer is selected from the group comprising: (i) a nitrogen-containing cationic functional group, or (ii) a phosphorus-containing cationic functional group; and/or the neutral ligand structure capable of coordinating with silver ions in the second polymer layer is selected from at least one of a thiol group, a carbonyl group, a pyridine ring, a pyrrole ring, a imidazole ring, a piperidine ring, or a thiazole ring; and/or in the third polymer layer, (i)the cationic functional group is selected from at least one of an amino group, a positively charged nitrogen heterocyclic structure, a quaternary ammonium cation, or a quaternary phosphine cation; and (ii) the anionic functional group is selected from at least one of a sulfonic acid group, a carboxylic acid group, or a phosphate group.

In some embodiments, (a) the first polymer layer is selected from at least one of a quaternized polyvinylpyridine layer, a quaternized polypyrrole layer, a quaternized polybenzimidazole layer, a quaternized polybenzothiazole layer, and a polyquaternized amphoteric ionic polymer layer; and/or (b) the second polymer layer is selected from at least one of : (i) neutral ligand-containing polymer layers comprising at least one of polyvinylpyridine, polypyrrole, polyvinylimidazole, thiol-functionalized polymers, carbonyl-functionalized polymers, or piperidine-functionalized polymers; (ii) sulfonic acid group-containing polymer layers comprising at least one of sulfonated polyvinylpyridine, sulfonated polypyrrole, sulfonated polybenzimidazole, sulfonated polybenzothiazole, polysulfobetaine, perfluorosulfonic acid polymers, sodium polystyrene sulfonate, or sodium polyethylene sulfonate; and/or (c) the third polymer layer is selected from at least one of cationic polymer layers, anionic polymer layers or amphoteric polymer layers: (i) the cationic polymer layers comprising at least one of polyethyleneimine, polyallylamine, polyacrylamide, polyaniline, poly(2-(dimethylamino)ethyl methacrylate), chitosan, polylysine, or perfluorosulfonic acid polymers; (ii) the anionic polymer layers comprising at least

one of polyacrylic acid, sodium polystyrene sulfonate, sodium polyethylene sulfonate, cellulose acetate, sodium alginate, hyaluronic acid, heparin, or polyphosphoric acid; (iii) the amphoteric polymer layers comprising polybetaine.

In some embodiments, a thickness of the sensor protective film is in the range from 5 μm to 50 μm.

In some embodiments, (a) the sensor protective film exhibits a water absorption rate of less than or equal to 20%; and (b) the sensor protective film exhibits a swelling ratio of less than or equal to 8%.

Another aspect of the present application may provide a method for preparing the sensor protective film, the method comprising: step (a) solidifying a first polymer film solution to form the first polymer layer, wherein the first polymer film solution comprises a nitrogen-containing cationic polymer, a phosphorus-containing cationic polymer, or a combination thereof; step (b) applying at least one of a second polymer film solution or a third polymer film solution onto a surface of the first polymer layer: (i) a second polymer film solution comprising a sulfonic acid group-containing polymer, a neutral ligand-containing polymer capable of coordinating with silver ions, or a combination thereof; or (ii) a third polymer film solution comprising an anionic group-containing polymer or a cationic group-containing polymer; onto a surface of the first polymer layer; and step (c) curing the applied solution(s) to form at least one of the second polymer layer or the third polymer layer, thereby obtaining the sensor protective film.

In some embodiments, the nitrogen-containing cationic polymer, the phosphorus-containing cationic polymer, or a combination thereof is present in the first polymer film solution in an amount of 5 wt% to 50 wt%, based on the total weight of the first polymer film solution; and/or (b) when applying the second polymer film solution: the sulfonic acid group-containing polymer, the silver-ion-coordinating neutral ligand-containing polymer, or a combination thereof is present in the second polymer film solution in an amount of 5 wt% to 50 wt%, based on the total weight of the second polymer film solution; and/or (c) when applying the third polymer film solution: the anionic group-containing polymer and/or the cationic group-containing polymer is present in the third polymer film solution in an amount of 5 wt% to 50 wt%, based on the total weight of the third polymer film solution.

In some embodiments, the first polymer film solution further comprises an epoxy-based crosslinking agent at a mass ratio of 1:100 to 3:20 relative to the nitrogen/phosphorus cationic group polymer; and/or the second polymer film solution further comprises an epoxy-based crosslinking agent at a mass ratio of 1:100 to 3:20 relative to the sulfonic acid group/neutral ligand structure polymer.

In some embodiments, (a) the nitrogen-containing cationic polymer, the phosphorus-containing cationic polymer, or a combination thereof is selected from at least one of quaternized polyvinylpyridine, quaternized polypyrrole, quaternized polybenzimidazole, quaternized polybenzothiazole, or polyquaternized amphoteric ionic polymer; and/or the epoxy-based crosslinking agent in each of the first polymer film solution and the second polymer film solution is independently selected from at least one of polyethylene glycol diglycidyl ether, poly(propylene glycol) diglycidyl ether, poly(dimethylsiloxane) diglycidyl ether, bisphenol A diglycidyl ether, 1,4-butanediol diglycidyl ether, tri(4-hydroxyphenyl) methane triglycidyl ether, and trimethylolpropane triglycidyl ether; and/or the sulfonic acid group-containing polymer, the silver-ion-coordinating neutral ligand-containing polymer, or a combination thereof is selected from at least one of polysulfobetaine, perfluorosulfonic acid polymer, sodium polystyrene sulfonate, sodium polyethylene sulfonate, polyvinylpyridine, polypyrrole, polyvinylimidazole, thiol polymer, carbonyl polymer, piperidine polymer, sulfonated polyvinylpyridine, sulfonated polypyrrole, sulfonated polybenzimidazole, and sulfonated polybenzothiazole; and/or the anionic group-containing polymer and/or the cationic group-containing polymer is selected from at least one of polyallylamine, polyethyleneimine, polyacrylamide, polyaniline, poly(2-(dimethylamino)ethyl methacrylate), polyacrylic acid, sodium polystyrene sulfonate, sodium polyethylene sulfonate, chitosan, cellulose acetate, sodium alginate, hyaluronic acid, heparin, polylysine, perfluorosulfonic acid polymer, polyphosphoric acid, or polybetaine.

Another aspect of the present application may provide an electrochemical sensor, comprising: a sensor substrate and; a sensor protective film disposed on the sensor substrate surface, wherein the sensor protective film is as mentioned before.

Additional features will be set forth in part in the description which follows, and in part will become apparent to those skilled in the art upon examination of the following and the accompanying drawings or may be learned by production or operation of the examples. The features of the present application may be realized and attained by practice or use of various aspects of the methodologies, instrumentalities, and combinations set forth in the detailed examples discussed below.

## BRIEF DESCRIPTION OF THE DRAWINGS

The present application is further illustrated in terms of exemplary embodiments. These exemplary embodiments are described in detail with reference to according to the drawings. These embodiments are non-limiting exemplary embodiments, in which like reference numerals represent similar structures, and wherein:

FIG. 1 depicts the infrared spectrum of the sensor protective film prepared in Example 1 of the present application;
FIG. 2 depicts the infrared spectrum of the sensor protective film prepared in Example 4 of the present application;

FIG. 3 is a schematic diagram of the apparatus used in the present application to determine the silver ion diffusion limitation capability of the sensor protective film via the diffusion cell method, wherein: A designates the first solution chamber, B designates the second solution chamber, and C designates the sensor protective film.

## DETAILED DESCRIPTION

In the following detailed description, numerous specific details are set forth by way of examples in order to provide a thorough understanding of the relevant disclosure. However, it should be apparent to those skilled in the art that the present application may be practiced without such details. In other instances, well-known methods, procedures, systems, components, and/or circuitry have been described at a relatively high level, without detail, in order to avoid unnecessarily obscuring aspects of the present application. Various modifications to the disclosed embodiments will be readily apparent to those skilled in the art, and the general principles defined herein may be applied to other embodiments and applications without departing from the spirit and scope of the present application. Thus, the present application is not limited to the embodiments shown, but is to be accorded the widest scope consistent with the claims.

The terminology used herein is for the purpose of describing particular example embodiments only and is not intended to be limiting. As used herein, the singular forms "a", "an", and "the" may be intended to include the plural forms as well, unless the context clearly indicates otherwise. It will be further understood that the terms "comprise", "comprises", and/or "comprising", "include", "includes", and/or "including", when used in this specification, specify the presence of stated features, integers, steps, operations, elements, and/or components, but do not preclude the presence or addition of one or more other features, integers, steps, operations, elements, components, and/or groups thereof.

It will be understood that the terms "system", "engine", "unit", "module", and/or "block" used herein are one method to distinguish different components, elements, parts, sections or assemblies of different levels in ascending order. However, the terms may be displaced by another expression if they achieve the same purpose.

It will be understood that when a unit, engine, module, or block is referred to as being "on", "connected to", or "coupled to", another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items. The terms "pixel" and "voxel" in the present application are used interchangeably to refer to an element of an image.

These and other features, and characteristics of the present application, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present application. It is understood that the drawings are not to scale.

For ease of understanding, a detailed description of the present application is provided hereinafter. It is to be understood, however, that the application may be embodied in various forms and is not limited to the embodiments set forth herein. Rather, these embodiments are provided to ensure thoroughness and completeness of the disclosure, enabling those skilled in the art to fully appreciate the scope of the application.

Unless otherwise defined, all technical and scientific terms used herein carry the same meaning as commonly understood by one of ordinary skill in the art to which this application pertains. The terminology employed in this specification is for describing specific embodiments only and is not intended to limit the application. As used herein, the term "and/or" includes any and all combinations of one or more of the associated listed items, meaning any single item, any combination of two or more items, or all items listed.

A reference electrode is a critical component of an electrochemical sensor, typically composed of an Ag/AgCl material. A primary requirement for the reference electrode is maintenance of stable potential; however, compositional or structural alterations may induce potential drift. Owing to the dissolution equilibrium: $AgCl(s) \rightleftharpoons Ag^{+}(aq) + Cl (aq)$, trace amounts of free silver ions reside within the AgCl layer. Driven by concentration gradients, these $Ag^{+}$ ions continuously diffuse into the external immersion medium. Leakage of $Ag^{+}$ from the AgCl layer, however, diminishes reference electrode stability, shortens sensor service life, and when the sensor is implanted in vivo presents biosafety risks due to the cytotoxicity of released silver ions. Conventional protective membranes used in sensors have proven inadequate to prevent $Ag^{+}$ leakage from the AgCl layer, thereby leading to compromised reference electrode stability, reduced sensor longevity, and suboptimal biosafety.

To address these challenges, attempts have been made to coat reference electrodes with polymer layers such as Nafion and polyurethane. However, the hydrophilic-hydrophobic microphase separation inherent in these materials fails to impede silver ion diffusion from hydrophilic domains into the external medium.

Currently, amphoteric ion membranes for vanadium redox flow batteries are known, formed by casting a mixed solution of a sulfonic acid group-containing first polymer and a second polymer which comprises an N-heterocyclic cation

or quaternary ammonium cation. These membranes leverage Donnan repulsion to reduce vanadium ion permeation while preserving electrical conductivity. Nonetheless, their fabrication method via direct mixing and curing suffers from two key deficiencies: (1) polymer interactions during curing often induce solid precipitation in the film solution; and (2) solvent evaporation during curing causes preferential precipitation of less-soluble polymers, leading to solid-liquid phase separation, local inhomogeneity, and compromised mechanical strength, thereby impairing cation-blocking performance.

As such, existing membrane technologies remain unable to effectively prevent $Ag^+$ leakage from AgCl layers in sensor protective applications, resulting in unstable reference electrodes, shortened sensor lifetimes, and inadequate biosafety.

The sensor protective film of the present application comprises a first polymer layer, and at least one additional layer, the first polymer layer and the at least one additional layer are arranged in a stacked configuration in layered configuration. The at least one additional layer is selected from the group comprising: a second polymer layer, or a third polymer layer. Specifically, the film structure may include: (1) a first polymer layer and a second polymer layer stacked in layers; (2) a first polymer layer and a third polymer layer stacked in layers; or (3) a first polymer layer, a second polymer layer, and a third polymer layer stacked in layers.

In the (3) film structure, the stacking sequence of the polymer layers may further include: (i) a first polymer layer, a second polymer layer, and a third polymer layer arranged in sequential order; (ii) a first polymer layer, a third polymer layer, and a second polymer layer arranged in sequential order; or (iii) a second polymer layer, a first polymer layer, and a third polymer layer arranged in sequential order.

The first polymer layer a first polymer layer comprising at least one polymer selected from the group comprising: (i) a nitrogen-containing cationic polymer, or (ii) a phosphorus-containing cationic polymer. The cationic polymer enhances the Donnan effect at the interface between the sensor protective film and $Ag^+$ ions. This effectively inhibits diffusion of free $Ag^+$ from the reference electrode's AgCl layer into the protective film driven by concentration gradients, thereby preventing $Ag^+$ leakage, stabilizing the reference electrode potential, and improving sensor biosafety.

The second polymer layer comprises sulfonic acid groups and/or neutral ligand structures capable of coordinating with silver ions. These components form stable complexes with $Ag^+$ through coordination bonding, enabling binding and immobilization of silver ions. This dualfunctionality creates a double-barrier mechanism to block $Ag^+$ permeation, further enhancing the sensor's biosafety profile.

The third polymer layer comprises anionic and/or cationic groups that establish electrostatic interactions with the first or second polymer layer, reinforcing the layered structure's stability. Such interactions improve the protective film's mechanical properties, including tensile strength and durability, thereby extending the sensor's operational lifespan.

Additionally, the layered composite structure of the present application mitigates two critical deficiencies of prior art membrane fabrication: (1) it avoids solid precipitation in the filmforming solution caused by inter-polymer interactions during direct mixing; and (2) it prevents solvent-evaporation-induced phase separation. Thereby eliminating local inhomogeneity and enhancing the protective film's overall uniformity and mechanical integrity.

The sensor protective film of the present application thus effectively prevents $Ag^+$ leakage from the reference electrode's AgCl layer, stabilizes reference electrode potential, improves sensor biosafety, and exhibits superior mechanical properties to extend sensor service life.

By laminating the second and/or third polymer layers onto the first polymer layer and varying the stacking sequence, different structural configurations of the protective film can be achieved each offering distinct $Ag^+$ diffusion suppression capabilities. To maximize $Ag^+$ blocking, the first polymer layer is preferably in direct contact with the sensor substrate or reference electrode surface, leveraging its cationic groups to resist positive charge migration, the second and/or third polymer layers onto the first polymer layer.

In a particularly preferred embodiment, the protective film comprises the first, second, and third polymer layers stacked in sequential order. This arrangement synergistically combines the Donnan effect from the first layer and chelation from the second layer to form a dual-barrier against $Ag^+$ permeation, while the third layer reinforces structural stability through electrostatic interactions, enhancing mechanical durability.

To improve interlayer adhesion, chemical bond connections are formed between the first and second polymer layers. More preferably, the second polymer layer is covalently bonded to each of the first polymer layer and the third polymer layer., significantly enhancing interlayer bonding strength, preventing delamination, and further improving the film's structural and mechanical properties.

In some embodiments, the nitrogen and/or phosphorus cationic groups are selected from at least one of a positively charged nitrogen heterocyclic structure, a quaternary ammonium cation, or a quaternary phosphine cation. The positively charged nitrogen heterocyclic structure and/or quaternary ammonium cations are preferred. Specifically, the positively charged nitrogen heterocyclic structure is selected from at least one of pyridine, pyrrole, imidazole, oxazole, isoxazole,thiazole, isothiazole, pyrazole, pyridazine, pyrimidine, pyrazine, piperazine,triazole, tetrazole, indole, quinoline, purine, aziridine, or azetidine.

In some embodiments, the neutral ligand structures capable of coordinating with silver ions are selected from at least one of a thiol group (-SH), carbonyl group (C=O), pyridine ring, pyrrole ring, imidazole ring, piperidine ring, or thiazole

ring, enhancing $Ag^+$ chelation and reducing permeation.

In some embodiments, in the third polymer layer, the cationic group is selected from at least one of an amino group, a positively charged nitrogen heterocyclic structure, a quaternary ammonium cation, or quaternary phosphine cation. Specifically, the cationic group is selected from at least one of a nitrogen heterocyclic structure or a quaternary ammonium cation. To augment Donnan repulsion between $Ag^+$ and the third polymer layer. The anionic group is selected from at least one of a sulfonic acid group ($-SO_3H$), carboxylic acid group (-COOH), or phosphate group ($-PO_4^{3-}$). With sulfonic acid groups being particularly preferred for strong $Ag^+$ binding.

In some embodiments, the first polymer layer is selected from at least one of a quaternized polyvinylpyridine layer, a quaternized polypyrrole layer, a quaternized polybenzimidazole layer, a quaternized polybenzothiazole layer, or a polyquaternized amphoteric ionic polymer layer.

In some embodiments, the second polymer layer is selected from at least one of (i) neutral ligand-containing polymer layers comprising at least one of polyvinylpyridine, polypyrrole, polyvinylimidazole, thiol-functionalized polymers, carbonyl-functionalized polymers, or piperidine-functionalized polymers; and (ii) sulfonic acid group-containing polymer layers comprising at least one of sulfonated polyvinylpyridine, sulfonated polypyrrole, sulfonated polybenzimidazole, sulfonated polybenzothiazole, polysulfobetaine, perfluorosulfonic acid polymers, sodium polystyrene sulfonate, or sodium polyethylene sulfonate.

In some embodiments, the third polymer layer is selected from at least one of: (i) cationic polymer layers comprising at least one of polyethyleneimine, polyallylamine, polyacrylamide, polyaniline, poly(2-(dimethylamino)ethyl methacrylate),chitosan, polylysine, or perfluorosulfonic acid polymers; (ii) anionic polymer layers comprising at least one of polyacrylic acid, sodium polystyrene sulfonate, sodium polyethylene sulfonate, cellulose acetate, sodium alginate, hyaluronic acid, heparin, or polyphosphoric acid; or (iii) amphoteric polymer layers comprising polybetaine.

In some embodiments, the sensor protective film has a thickness ranging from 5 $\mu$m to 50 $\mu$m. Such thickness ensures the protective film exhibits excellent mechanical properties including tensile strength and flexibility while optimizing its coating uniformity on the reference electrode surface or sensor substrate surface.

In some embodiments, the sensor protective film, as well as each of its constituent layers (i.e., the first, second, and third polymer layers), has a water absorption rate $\leq$ 20% and a swelling degree $\leq$ 8%. Specifically: the first polymer layer demonstrates a water absorption rate $\leq$ 20% and swelling degree $\leq$ 8%; the second polymer layer demonstrates a water absorption rate $\leq$ 20% and swelling degree $\leq$ 8%; the third polymer layer demonstrates a water absorption rate $\leq$ 20% and swelling degree $\leq$ 8%. These specifications for water absorption and swelling degree enhance the protective film's structural stability under aqueous environments and strengthen its adhesion to the underlying electrode or substrate, thereby minimizing delamination risks and maintaining long-term functional integrity.

The present application further provides a method for preparing the sensor protective film, comprising: step a: Solidify a first polymer film solution to form a first polymer layer, wherein the first polymer film solution comprises a polymer containing nitrogen and/or phosphorus cationic groups; step b: Apply a second polymer film solution and/or a third polymer film solution onto the surface of the first polymer layer and cure the applied solution(s) to form a second polymer layer and/or a third polymer layer, thereby obtaining the sensor protective film.

Specifically: the second polymer film solution comprises a polymer containing sulfonic acid groups and/or a neutral ligand structure capable of coordinating with silver ions; the third polymer film solution comprises a polymer containing anionic and/or cationic groups.

The step a further comprising: the first polymer film solution has a mass fraction of nitrogen and/or phosphorus cationic group-containing polymer of 5% to 50%. This concentration range ensures the formed first polymer layer is rich in cationic groups, thereby enhancing the Donnan repulsion effect between the first polymer layer and $Ag^+$ ions to more effectively inhibit silver ion diffusion.

The first polymer film solution may further include an epoxy-based crosslinking agent, with a mass ratio of epoxy-based crosslinking agent to nitrogen/phosphorus cationic group-containing polymer of 1:100 to 3:20. This ratio promotes the formation of uniformly distributed chemical crosslinking points within the first polymer layer during curing, which: (1) suppresses water absorption and swelling of the first polymer layer; (2) prevents delamination from other polymer layers due to excessive water-induced deformation; (3) provides epoxy groups that form chemical bonds via ring-opening reactions with positively charged nitrogen heterocycles and/or amino groups in the second/third polymer layers, enabling interlayer chemical bond connections.

The nitrogen and/or phosphorus cationic group-containing polymer is selected from at least one of a quaternized polyvinylpyridine polymer, a quaternized polypyrrole polymer, a quaternized polybenzimidazole polymer, a quaternized polybenzothiazole polymer, or a polyquaternized amphoteric ionic polymer.

The epoxy-based crosslinking agent in the first polymer film solution is selected from at least one of polyethylene glycol diglycidyl ether, poly(propylene glycol) diglycidyl ether, poly(dimethylsiloxane) diglycidyl ether, bisphenol A diglycidyl ether, 1,4-butanediol diglycidyl ether, tri(4-hydroxyphenyl) methane triglycidyl ether, or trimethylolpropane triglycidyl ether. Specifically, the nitrogen and/or phosphorus cationic group-containing polymer is selected from at least one of polyethylene glycol diglycidyl ether, triethyleneglycol diglycidyl ether, or trimethylolpropane triglycidyl ether.

In some embodiments, the first polymer film solution is applied to a substrate surface via blade coating, spin coating, spray coating, or dip coating, followed by substrate removal to obtain the first polymer layer. Suitable substrates comprises polystyrene or polytetrafluoroethylene.

In some embodiments, the first polymer film solution is poured into a mold, solidified, and then the solidified film is peeled from the mold to form the first polymer layer.

In some embodiments, curing in step a is performed via vacuum drying at a temperature of 30°C to 100°C for 12 hours to 48 hours, ensuring complete solvent evaporation and crosslinking.

In some embodiments, step S2 further comprising: the second polymer film solution has a mass fraction of sulfonic acid group- and/or silver-ion-coordinating neutral ligand structure-containing polymer of 5% to 50%. This concentration range ensures the formed second polymer layer contains sufficient coordination groups to immobilize $Ag^+$ ions diffusing from the AgCl layer, thereby preventing ion leakage.

The sulfonic acid group- and/or silver-ion-coordinating neutral ligand structure-containing polymer is selected from at least one of polysulfobetaine, perfluorosulfonic acid polymer, sodium polystyrene sulfonate, sodium polyethylene sulfonate, polyvinylpyridine, polypyrrole, polyvinylimidazole, thiol polymer, carbonyl polymer, piperidine polymer, sulfonated polyvinylpyridine, sulfonated polypyrrole, sulfonated polybenzimidazole, or sulfonated polybenzothiazole.

Specifically, the sulfonic acid group- and/or silver-ion-coordinating neutral ligand structure-containing polymer is selected from at least one of sulfonated polyvinylpyridine, sulfonated polypyrrole, sulfonated polybenzimidazole, or sulfonated polybenzothiazole. This dual functionality ensures strong $Ag^+$ binding while providing anionic groups for electrostatic interaction with adjacent layers, enhancing interlayer adhesion.

In some embodiments, the second polymer film solution may further include an epoxy-based crosslinking agent at a mass ratio of 1:100 to 3:20 (crosslinking agent to polymer). This ratio: (1) mitigates water-induced deformation and delamination risks; (2) provides epoxy groups that undergo ring-opening reactions with nitrogen-containing cationic groups in the first/third layers, forming covalent interlayer bonds; (3) ensures uniform crosslinking to maintain film integrity during coating on electrode/substrate surfaces.

Specifically, the epoxy-based crosslinking agents used in the second layer are consistent with those described for the first layer.

In some embodiments, in step b, the second polymer film solution and the third polymer film solution are sequentially applied to the same surface of the first polymer layer and cured to form the second polymer layer and the third polymer layer. When the first polymer film solution comprises a polymer containing nitrogen and/or phosphorus-containing cationic groups, the second polymer film solution comprises an epoxy-based crosslinking agent, a sulfonic acid group-containing polymer, and/or a polymer containing neutral ligand structures capable of coordinating with silver ions, and the third polymer film solution comprises a polymer containing cationic groups (e.g., amino groups, positively charged nitrogen heterocyclic structures, quaternary ammonium cations), the following occurs:

During the curing process, the epoxy crosslinking agent in the second polymer film solution can chemically react with nitrogen atoms in both the nitrogen and/or phosphorus-containing cationic group polymer and the cationic group-containing polymer (e.g., amino groups, positively charged nitrogen heterocyclic structures, quaternary ammonium cations) via ring-opening reactions to form chemical bonds, achieving chemical bond connections between the second polymer layer and both the first polymer layer and the third polymer layer.

Simultaneously, the second polymer layer can also achieve physical adsorption connections with the first and third polymer layers through electrostatic interactions between them. Thus, under the dual action of chemical bond connections and physical adsorption connections, the bonding between layers is further enhanced, preventing delamination and improving the mechanical properties of the sensor protective film.

In some embodiments, the third polymer film solution has a mass fraction of anionic group-containing polymer and/or the cationic group-containing polymer of 5% to 50%.

In some embodiments, the anionic group-containing polymer and/or the cationic group-containing polymer is selected from at least one of polyallylamine, polyethyleneimine, polyacrylamide, polyaniline, poly(2-(dimethylamino)ethyl methacrylate), polyacrylic acid, sodium polystyrene sulfonate, sodium polyethylene sulfonate, chitosan, cellulose acetate, sodium alginate, hyaluronic acid, heparin, or polylysine.

Specifically, the anionic group-containing polymer and/or the cationic group-containing polymer is selected from at least one of polyacrylic acid, chitosan, or sodium alginate. Which enables the formation of a sensor protective film with a more stable structure.

In some embodiments, solvents for the first, second, and third polymer film solutions are independently selected from a first solvent or a mixed solvent of the first solvent and water, where the first solvent includes at least one of methanol, ethanol, propanol, butanol, acetone, tetrahydrofuran, dimethyl sulfoxide, of 1,4-dioxane.

Preferably, the mixed solvent is a mixture of a first solvent and water, wherein the volume ratio of the first solvent to water in the mixed solvent is 1:6 to 6:1.

In some embodiments, curing of the second and/or third polymer layers in step b follows the same conditions as step a.

Unlike traditional single-step mixing and curing, the layered fabrication method of the present application eliminates polymer phase separation and solid precipitation during film formation. This results in a sensor protective film with superior uniformity, interlayer adhesion, and mechanical properties, directly addressing the deficiencies of prior art membrane technologies.

In some embodiments, the present application further provides an electrochemical sensor comprising a sensor substrate and a sensor protective film disposed on the sensor substrate surface, wherein the sensor protective film is as described above. The electrochemical sensor exhibits high operational stability, extended service life, and enhanced biosafety.

For practical applications, the sensor protective film may be directly coated onto the reference electrode surface or, for process simplicity, onto the outer surface of the sensor substrate.

In some embodiments, the sensor substrate surface bears reactive functional groups, preferably hydroxyl (-OH) and/or amino ($-NH_2$) groups. When a first or second polymer film solution is applied to the substrate surface and cured, epoxy groups from the epoxy-based crosslinking agent in the solution undergo ring-opening reactions with the substrate's hydroxyl or amino groups, forming covalent bonds. This establishes chemical bond connections between the polymer layer and the substrate, enhancing adhesion, enabling long-term $Ag^+$ retention, and extending sensor lifespan.

In some embodiments, reactive groups on the sensor substrate surface may be introduced via plasma treatment or chemical vapor deposition (CVD) technology, ensuring uniform surface modification for robust interlayer bonding.

In some embodiments, the sensor protective film is applied to the sensor substrate surface using the layered fabrication method described herein. For example: a first polymer film solution is applied to the substrate via spraying, dip-coating, or spin-coating and cured to form the first polymer layer; a second polymer film solution is applied to the first layer-coated substrate and cured to form the second polymer layer; a third polymer film solution is applied to the bilayer-coated substrate and cured to form the third polymer layer, resulting in the complete electrochemical sensor structure.

The stacking sequence of polymer layers in contact with the substrate, either the first or second layer, may be adjusted to optimize performance; direct contact between the first polymer layer and the substrate is preferred to maximize Donnan repulsion at the electrode interface.

In some embodiments, the sensor substrate surface comprises a sequentially arranged PET (polyethylene terephthalate) or PI (polyimide) substrate, a carbon paste conductive coating, and a functional film layer, providing a stable base for protective film deposition.

The sensor protective film, its preparation method, and applications will be further illustrated by the following specific embodiments. However, those skilled in the art will understand that the following embodiments are provided for illustrative purposes only and should not be construed as limiting the scope of the present invention. Where specific conditions are not noted in the examples, the procedures are conducted under conventional conditions or conditions recommended by the manufacturer. Reagents or equipment used without indicating the manufacturer are all commercially available conventional products.

In the examples and comparative examples of the present invention, the thickness of the sensor protective film is measured using a micrometer. Additionally, the infrared spectra of Example 1 and Example 4 of the present invention are detected by a Fourier transform infrared (FTIR) spectrometer.

Furthermore, the first polymers having the structures shown in Formula (1), Formula (2), and Formula (3) in the examples and comparative examples of the present invention are

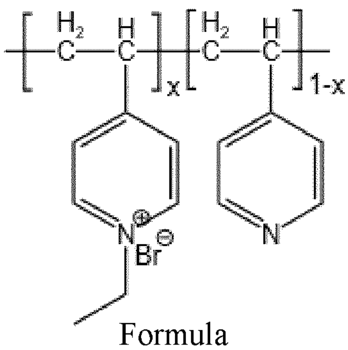

Formula (1)

,

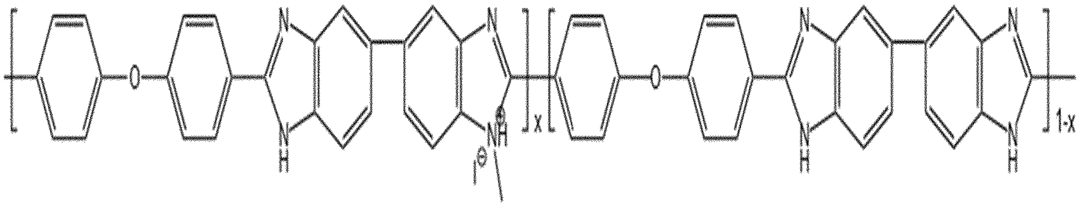

Formula (2)

,

and

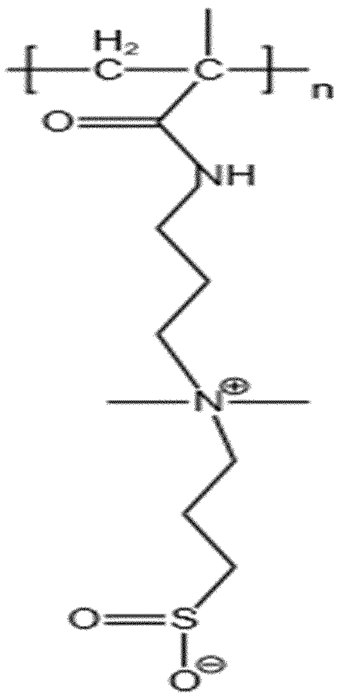

Formula (3)

Wherein, in Formula (1) and Formula (2), x denotes the molar fraction of the structural unit relative to total units; in Formula (3), n denotes the degree of polymerization.

Example 1

Synthesis of First Polymer (Quaternized Poly(4-vinylpyridine)):10 g of poly(4-vinylpyridine) (CAS No. 9017-40-7, Aladdin) was reacted with 5.2 g of ethyl bromide (CAS No. 74-96-4) at 100°C for 24 hours to yield quaternized poly(4-vinylpyridine) with a 50% degree of ethylation, corresponding to the first polymer having the structure of Formula (1) (where x = 0.5).

Synthesis of Second Polymer (Sulfonated Poly(4-vinylpyridine)):10 g of poly(4-vinylpyridine) (CAS No. 9017-40-7, Aladdin) was reacted with 2.6 g of 1,4-butanesultone (CAS No. 1633-83-6, Aladdin) at 80°C for 24 hours to produce sulfonated poly(4-vinylpyridine) with a 20% degree of sulfonation, serving as the second polymer.

The first polymer having the structure shown in Formula (1) is mixed with methanol to prepare a first polymer film solution, wherein the mass fraction of the first polymer in the first polymer film solution is 5%. Sulfonated poly(4-vinylpyridine) with a sulfonation degree of 20% is mixed with methanol to prepare a second polymer film solution, wherein the mass fraction of sulfonated poly(4-vinylpyridine) in the second polymer film solution is 15%. Polyethyleneimine (CAS No.: 9002-98-6, Aladdin) is mixed with ethanol to prepare a third polymer film solution, wherein the mass fraction of polyethyleneimine in the third polymer film solution is 20%.

The first polymer film solution obtained above is sprayed onto the surface of a polystyrene substrate and vacuum-dried at 40°C for curing to form a first polymer layer. Subsequently, the second polymer film solution is sprayed onto the

surface of the first polymer layer and vacuum-dried at 40°C for curing to form a second polymer layer. Next, the third polymer film solution is sprayed onto the surface of the second polymer layer and vacuum-dried at 40°C for curing to form a third polymer layer. The polystyrene substrate is then removed to obtain a sensor protective film with a thickness of approximately 40 μm.

Figure 1 exhibits strong absorption peaks at 3300 $cm^{-1}$ to3500 $cm^{-1}$ attributed to primary amine groups in the third polymer (polyethyleneimine) and aliphatic C-H stretching vibrations at 2931 $cm^{-1}$ and 2817 $cm^{-1}$ (methylene groups in polyethyleneimine). Characteristic pyridine ring absorption peaks at 1596 $cm^{-1}$, 1557 $cm^{-1}$, 1416 $cm^{-1}$, and 820 $cm^{-1}$ confirm the presence of the first and second polymers. A distinct peak at 1039 $cm^{-1}$ corresponds to the sulfonic acid group stretching vibration in the second polymer. These spectral data confirm the successful fabrication of the sensor protective film, which comprises the first, second, and third polymer layers in layered configuration.

Example 2

The procedure of Example 2 is identical to Example 1, except that the third polymer layer is omitted. Specifically: the second polymer film solution was spray-coated onto the first polymer layer-coated polystyrene substrate; the coated substrate was vacuum-dried and cured at 40°C to form the second polymer layer; the polystyrene substrate was removed, yielding a sensor protective film with a thickness of approximately 20 μm.

Example 3

Example 3 differs from Example 1 solely in the absence of the second polymer layer. The modified procedure is as follows: the third polymer film solution was spray-coated onto the first polymer layer-coated polystyrene substrate; the coated substrate was vacuum-dried and cured at 40°C to form the third polymer layer; the polystyrene substrate was removed, resulting in a sensor protective film with a thickness of approximately 25 μm.

Example 4

Example 4 is identical to Example 1, except that the first polymer film solution includes polyethylene glycol diglycidyl ether (CAS No. 39443-66-8, Aladdin) as an epoxy-based crosslinking agent at a mass fraction of 0.05%. All other conditions remain unchanged, yielding a sensor protective film with a thickness of approximately 41 μm.

As depicted in Figure 2, the third polymer polyethyleneimine exhibits two distinct strong absorption peaks at 3300 $cm^{-1}$ to 3500 $cm^{-1}$ attributed to primary amine groups, alongside aliphatic C-H stretching vibrations at 2935 $cm^{-1}$ and 2840 $cm^{-1}$ corresponding to methylene groups in polyethyleneimine. Characteristic absorption peaks for the pyridine structure in the first and second polymers are observed at 1553 $cm^{-1}$, 1411 $cm^{-1}$, and 820 $cm^{-1}$, while a distinct peak at 1036 $cm^{-1}$ arises from the sulfonic acid group stretching vibrations in the second polymer. Notably, the 1596 $cm^{-1}$ pyridine characteristic peak present in Figure 1 (Example 1) is absent in Example 4. This spectral change is attributed to the inclusion of polyethylene glycol diglycidyl ether in the first polymer film solution. These infrared spectral data confirm the successful fabrication of the sensor protective film in Example 4, which comprises the first, second, and third polymer layers stacked in sequence as claimed.

Example 5

The sole distinction between Example 5 and Example 1 lies in the inclusion of polyethylene glycol diglycidyl ether within the second polymer film solution. Specifically, the second polymer film solution contains polyethylene glycol diglycidyl ether at a mass fraction of 2%. All other conditions remain identical to those of Example 1, resulting in the formation of a sensor protective film with an approximate thickness of 44 μm.

Example 6

Example 6 differs from Example 5 only in that, in preparing the second polymer film solution, trimethylethylene triglycidyl ether (CAS No.: 36366-26-4, Sigma) is used instead of polyethylene glycol diglycidyl ether, and the mass fraction of trimethylethylene triglycidyl ether in the second polymer film solution is 2%. The remaining conditions are the same, resulting in a sensor protective film with a thickness of approximately 43 μm.

Example 7

The only difference between Example 7 and Example 1 is that, during the preparation of the second polymer film solution, sodium polystyrene sulfonate (CAS No. 25704 - 18 - 1, Aladin) is utilized in place of sulfonated poly(4 -

vinylpyridine). The mass fraction of sodium polystyrene sulfonate in the second polymer film solution is 10%, and all other conditions remain unchanged. As a result, a sensor protective film with an approximate thickness of 50 μm is obtained.

Example 8

Example 8 differs from Example 1 only in that 10 g of polybenzimidazole (CAS No.: 25928-81-8, Macklin) is reacted with 1.1 g of methyl iodide (CAS No.: 74-88-4, Aladdin) at 60°C for 24 hours to prepare a quaternized polybenzimidazole with a methylation degree of 30%, i.e., the first polymer having the structure shown in Formula (2) (where x = 0.3).

The first polymer with the structure shown in Formula (2) was dissolved in methanol to prepare the first polymer film solution, in which the mass fraction of quaternary ammonium polybenzimidazole was 15%. All other conditions remained the same as in Example 1, resulting in the formation of a sensor protective film with an approximate thickness of 50 μm.

Example 9

The only distinction between Example 9 and Example 1 is that 1 g of [2-(methylacryloyoxy)ethyl]dimethyl-(3-propyl sulfonate)ammonium hydroxide (CAS No. 3637 - 26 - 1, Aladine) was polymerized via free radical polymerization at 70 °C to produce a polyquaternary ammonium amphoteric polymer, corresponding to the first polymer having the structure of Formula (3) (with a degree of polymerization n = 100 as determined by GPC).

The first polymer with the structure shown in Formula (3) was dissolved in water to prepare the first polymer film solution, in which the mass fraction of the polyquaternary ammonium amphoteric ionic polymer was 15%. All other conditions were identical to those of Example 1, yielding a sensor protective film with an approximate thickness of 15 μm.

Example 10

Example 10 differs from Example 1 only in that, in preparing the second polymer film solution, poly(4-vinylpyridine) (CAS No.: 9017-40-7, Aladdin) is used instead of sulfonated poly(4-vinylpyridine), and the mass fraction of poly(4-vinylpyridine) in the second polymer film solution is 10%. In preparing the third polymer film solution, polyacrylic acid (CAS No.: 9003-01-4, Aladdin) is used instead of polyethyleneimine, and the mass fraction of polyacrylic acid in the third polymer film solution is 15%. The remaining conditions are the same, resulting in a sensor protective film with a thickness of approximately 30 μm.

Comparison Example 1

A mixed solution is prepared by combining the first polymer having the structure of Formula (1) (x = 0.5), sulfonated poly(4-vinylpyridine), polyethyleneimine, and ethanol. The mixed solution contains: 5% mass fraction of the first polymer, 15% mass fraction of sulfonated poly(4-vinylpyridine), and 20% mass fraction of polyethyleneimine. The mixed solution is spray-coated onto a polystyrene substrate and vacuum-dried at 40°C. After drying, the polystyrene substrate is removed, yielding a sensor protective film with an approximate thickness of 20 μm.

Comparison Example 2

A mixed solution is prepared by combining the first polymer having the structure of Formula (1) (x = 0.5), sulfonated poly(4-vinylpyridine), and ethanol, excluding polyethyleneimine. The mixed solution contains: 5% mass fraction of the first polymer, 15% mass fraction of sulfonated poly(4-vinylpyridine). The mixed solution is spray-coated onto a polystyrene substrate and vacuum-dried at 40°C. After drying, the polystyrene substrate is removed, resulting in a sensor protective film with an approximate thickness of 10 μm.

Comparison Example 3

A mixed solution is prepared by combining the first polymer having the structure of Formula (1) (x = 0.5), sulfonated poly(4-vinylpyridine), polyethyleneimine, polyethylene glycol diglycidyl ether, and ethanol. The mixed solution contains: 5% mass fraction of the first polymer, 15% mass fraction of sulfonated poly(4-vinylpyridine), 20% mass fraction of polyethyleneimine.

The mixed solution is spray-coated onto a polystyrene substrate and vacuum-dried/cured at 40°C. After drying, the polystyrene substrate is removed, yielding a sensor protective film with an approximate thickness of 11 μm.

Performance testing was conducted on the sensor protective films prepared in Example 1 to Example 13, with

test methods as follows:

Water absorption rate: the sensor protective film was immersed in 0.01M PBS buffer (pH 7.4, 37°C simulating human body fluid environment). The initial mass ($m_1$) before immersion and the mass ($m_2$) after 2 hours of immersion were measured. The water absorption rate was calculated using the formula:

$$\text{Water absorption rate} = m_1/m_2 \times 100\%$$

Swelling degree: the sensor protective film was immersed in PBS buffer (pH 7.4, 37°C). The initial thickness ($d_1$) before immersion and the thickness ($d_2$) after 2 hours of immersion were measured. The swelling degree was calculated using the formula:

$$\text{Swelling degree} = d_1/d_2 \times 100\%$$

Tensile strength was tested in accordance with GB 13022-1991 (Standard Test Method for Tensile Properties of Plastics).

Silver ion diffusion limitation capacity: the diffusion cell method was employed to evaluate the silver ion diffusion limitation capacity, with the experimental setup illustrated in Figure 3. Chamber A was filled with a 100 mM silver nitrate solution, while Chamber B contained 0.1 M phosphate-buffered saline (PBS) at a pH of 7.4. Following an incubation period of one hour, the concentration of silver ions in Chamber B was determined using inductively coupled plasma (ICP) spectroscopy.

Table 1

| | Water Absorption Rate /% | Swelling Degree /% | Tensile Strength /MPa | Silver Ion Concentration /mM | Silver Ion Diffusion Coefficient /$cm^2 \cdot s^{-1}$ |
|---|---|---|---|---|---|
| Example 1 | 16.3 | 6.2 | 955 | 1.3 | $5.34\times10^{-8}$ |
| Example 2 | 12.4 | 5 | 908 | 2.85 | $5.95\times10^{-8}$ |
| Example 3 | 14 | 5.1 | 850 | 3.66 | $9.63\times10^{-8}$ |
| Example 4 | 10.5 | 4.4 | 1116 | 1.1 | $4.62\times10^{-8}$ |
| Example 5 | 11.1 | 4.6 | 1204 | 0.98 | $4.41\times10^{-8}$ |
| Example 6 | 10.8 | 4 | 1358 | 0.89 | $3.91\times10^{-8}$ |
| Example 7 | 15.6 | 6 | 923 | 2.45 | $1.27\times10^{-7}$ |
| Example 8 | 13.7 | 5.2 | 980 | 1.22 | $6.26\times10^{-8}$ |
| Example 9 | 20 | 8 | 786 | 1.51 | $7.77\times10^{-8}$ |
| Example 10 | 18.2 | 7 | 921 | 4.8 | $1.53\times10^{-7}$ |
| Comparison Example 1 | 31.9 | 13.5 | 145 | 12.5 | $2.92\times10^{-7}$ |
| Comparison Example 2 | 27.7 | 11.8 | 310 | 25.6 | $3.64\times10^{-7}$ |
| Comparison Example 3 | 25.8 | 10.5 | 501 | 16.6 | $2.25\times10^{-7}$ |

Example 11

Utilizing the first, second, and third polymer film solutions prepared according to the method of Example 1: the sensor substrate was immersed in the first polymer film solution, withdrawn, and vacuum-dried at 50°C to form the first polymer layer on the substrate surface. The sensor substrate coated with the first polymer layer was immersed in the second polymer film solution, withdrawn, and vacuum-dried at 50°C to form the second polymer layer on the first polymer layer surface. The sensor substrate bearing the first and second polymer layers was immersed in the third polymer film solution, withdrawn, and vacuum-dried at 50°C to form the third polymer layer on the second polymer layer surface, resulting in an electrochemical sensor with a sensor protective film on its surface. The sensor protective film exhibited an approximate thickness of 28 μm.

Example 12

Utilizing the first and second polymer film solutions prepared as in Example 1: the sensor substrate was immersed in the first polymer film solution, withdrawn, and vacuum-dried at 50°C to form the first polymer layer on the substrate surface. The substrate coated with the first polymer layer was immersed in the second polymer film solution, withdrawn, and vacuum-dried at 50°C to form the second polymer layer on the first layer surface, resulting in an electrochemical sensor with a surface-applied sensor protective film. The sensor protective film had an approximate thickness of 14 μm.

Example 13

Utilizing the first, second, and third polymer film solutions prepared as in Example 4: the sensor substrate was immersed in the first polymer film solution, withdrawn, and vacuum-dried at 50°C to form the first polymer layer on the substrate surface. The substrate with the first polymer layer was immersed in the second polymer film solution, withdrawn, and vacuum-dried at 50°C to form the second polymer layer on the first layer surface. The substrate bearing the first and second polymer layers was immersed in the third polymer film solution, withdrawn, and vacuum-dried at 50°C to form the third polymer layer on the second layer surface, yielding an electrochemical sensor with a surface-coated sensor protective film. The sensor protective film exhibited an approximate thickness of 28 μm.

Example 14

Utilizing the first, second, and third polymer film solutions prepared as in Example 5: the sensor substrate was immersed in the first polymer film solution, withdrawn, and vacuum-dried at 50°C to form the first polymer layer on the substrate surface. The substrate with the first polymer layer was immersed in the second polymer film solution, withdrawn, and vacuum-dried at 50°C to form the second polymer layer on the first layer surface. The substrate with the first and second polymer layers was immersed in the third polymer film solution, withdrawn, and vacuum-dried at 50°C to form the third polymer layer on the second layer surface, resulting in an electrochemical sensor with a surface-applied sensor protective film. The sensor protective film had an approximate thickness of 30 μm.

Example 15

Example 15 differs from Example 13 only in that the sensor substrate surface was treated via plasma treatment to introduce hydroxyl and amino groups. This functionalized substrate bearing surface hydroxyl and amino groups replaced the untreated substrate of Example 13, with all other conditions remaining identical. The resulting electrochemical sensor featured a surface-applied sensor protective film with an approximate thickness of 30 μm.

Comparison Example 4

Using the mixed polymer film solution prepared according to Comparison Example 1: the sensor substrate was immersed in the mixed film solution, withdrawn, and vacuum-dried at 50°C to form a sensor protective film on the substrate surface.This process yielded an electrochemical sensor with a surface-applied sensor protective film having an approximate thickness of 9 μm.

Comparison Example 5

Using the mixed polymer film solution prepared according to Comparison Example 2: the sensor substrate was immersed in the mixed film solution, withdrawn, and vacuum-dried at 50°C to form a sensor protective film on the substrate surface.This process resulted in an electrochemical sensor with a surface-applied sensor protective film having an approximate thickness of 5 μm.

Comparison Example 6

Using the mixed polymer film solution prepared according to Comparison Example 3: the sensor substrate was immersed in the mixed film solution, withdrawn, and vacuum-dried at 50°C to form a sensor protective film on the substrate surface.This process produced an electrochemical sensor with a surface-applied sensor protective film having an approximate thickness of 8 μm.

The electrochemical sensors with surface-applied sensor protective films (Example 11 to Example 15 and Comparison Example 4 to Comparison Example 6) and the electrochemical sensor without a protective film (designated

as Comparison Example 7) were tested as follows, with results summarized in Table 2:

Cross-Cut Adhesion Test (Hundred-Grid Test): Adhesion of the sensor protective film to the sensor substrate surface was evaluated in accordance with GB 9286-1998 (Paints and Varnishes Cross-Cut Test for Adhesion). The hundred-grid test grade is inversely proportional to adhesion: a lower grade indicates stronger adhesion between the protective film and the substrate surface.

Silver ion diffusion limitation test: the test electrochemical sensor's surface area was extracted with PBS (pH 7.4, 0.1M) at a surface area-to-extract volume ratio of 5 $cm^2$:1 mL for 12 hours. The silver ion concentration in the extract was measured using inductively coupled plasma (ICP) spectroscopy to determine the protective film's capability to limit silver ion diffusion.

Table 2

| | Hundred-Grid Test Grade | Silver Ion Concentration /mM |
|---|---|---|
| Example 11 | 2 | $7.41\times10^{-4}$ |
| Example 12 | 2 | $1.02\times10^{-3}$ |
| Example 13 | 1 | $3.71\times10^{-4}$ |
| Example 14 | 1 | $3.71\times10^{-4}$ |
| Example 15 | 0 | $2.78\times10^{-4}$ |
| Comparison Example 4 | 4 | $3.06\times10^{-3}$ |
| Comparison Example 5 | 4 | $3.43\times10^{-3}$ |
| Comparison Example 6 | 3 | $2.59\times10^{-3}$ |
| Comparison Example 7 | / | $1.11\times10^{-2}$ |

The data in Table 1 and Table 2 demonstrate that the sensor protective film of the present application effectively mitigates $Ag^+$ leakage from the reference electrode's AgCl layer, stabilizes reference electrode potential, enhances sensor biosafety, and exhibits superior mechanical properties to extend sensor operational lifespan.

Specifically, results from Example 1, Example 2, Example 4, Example 1 1 to Example 15, and corresponding Comparison Example 1 to Comparison Example 6 indicate that the layered composite fabrication process improves the sensor protective film's mechanical properties including tensile strength and swelling resistance and augments its ability to limit silver ion diffusion. This dual improvement directly contributes to prolonged sensor service life and reduced $Ag^+$ permeation.

Data from Example 13 and Example 15 further show that epoxy-based crosslinking agents facilitate covalent bond formation with hydroxyl/amino groups on the sensor substrate surface via ring-opening reactions. This chemical bonding mechanism establishes robust interlayer adhesion, significantly enhancing the protective film's structural integrity and resistance to delamination under physiological conditions.

All technical features disclosed in the foregoing embodiments may be combined in any technically feasible manner. For brevity, this specification does not explicitly describe every possible combination of these technical features; however, any combination lacking internal contradiction shall be deemed to fall within the scope of this disclosure.

The embodiments described herein represent only a subset of the present application's possible implementations. While the description is specific and detailed, it should not be interpreted as limiting the application's scope. It is noted that those skilled in the art may make various variations and improvements without departing from the application's core concept, all of which are encompassed within the application's protective scope. Accordingly, the scope of the present application shall be defined exclusively by the appended claims.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present application, various features are sometimes grouped together in a single embodiment, figure, or description thereof for the purpose of streamlining the disclosure aiding in the understanding of one or more of the various embodiments. This method of

disclosure, however, is not to be interpreted as reflecting an intention that the claimed subject matter requires more features than are expressly recited in each claim. Rather, claim subject matter lie in less than all features of a single foregoing disclosed embodiment.

In some embodiments, the numbers expressing quantities or properties used to describe and claim certain embodiments of the application are to be understood as being modified in some instances by the term "about", "approximate", or "substantially". For example, "about", "approximate", or "substantially" may indicate a certain variation (e.g., ±1%, ±5%, ±10%, or ±20%) of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable. In some embodiments, a classification condition used in classification or determination is provided for illustration purposes and modified according to different situations. For example, a classification condition that "a value is greater than the threshold value" may further include or exclude a condition that "the probability value is equal to the threshold value."

## Claims

1. A sensor protective film, wherein the film comprising:
a first polymer layer and at least one additional layer, the first polymer layer and the at least one additional layer are arranged in a stacked configuration ;

    (a) the first polymer layer comprising at least one cationic functional group selected from the group comprising: (i) a nitrogen-containing cationic functional group, or (ii) a phosphorus-containing cationic functional group; and
    (b) the additional layer selected from at least one of a second polymer layer, or a third polymer layer,

        wherein (i) the second polymer layer comprising at least one polymer selected from the group comprising: a sulfonic acid group-containing polymer, or a silver-ion-coordinating neutral ligand-containing polymer,
        (ii) the third polymer layer comprising at least one of an anionic functional group or a cationic functional group.

2. The sensor protective film of claim 1, wherein the sensor protective film comprises the first polymer layer, the second polymer layer, and the third polymer layer sequentially stacked.

3. The sensor protective film of claim 2, wherein the second polymer layer is covalently bonded to each of the first polymer layer and the third polymer layer.

4. The sensor protective film of claims 1, wherein:

    (a) the cationic functional group in the first polymer layer is selected from at least one of a positively charged nitrogen heterocyclic structure, a quaternary ammonium cation, or a quaternary phosphine cation; the cationic functional group in the first polymer layer is selected from the group comprising: (i) a nitrogen-containing cationic functional group, or (ii) a phosphorus-containing cationic functional group; and/or
    (b) the neutral ligand structure capable of coordinating with silver ions in the second polymer layer is selected from at least one of a thiol group, a carbonyl group, a pyridine ring, a pyrrole ring, a imidazole ring, a piperidine ring, or a thiazole ring; and/or
    (c) in the third polymer layer,

        (i) the cationic functional group is selected from at least one of an amino group, a positively charged nitrogen heterocyclic structure, a quaternary ammonium cation, or a quaternary phosphine cation; and
        (ii) the anionic functional group is selected from at least one of a sulfonic acid group, a carboxylic acid group, or a phosphate group.

5. The sensor protective film of claim 4, wherein:

    (a) the first polymer layer is selected from at least one of a quaternized polyvinylpyridine layer, a quaternized polypyrrole layer, a quaternized polybenzimidazole layer, a quaternized polybenzothiazole layer, or a polyquaternized amphoteric ionic polymer layer; and/or
(b) the second polymer layer is selected from at least one of neutral ligand-containing polymer layers or sulfonic acid group-containing polymer layers,

wherein (i) the neutral ligand-containing polymer layers comprising at least one of polyvinylpyridine, polypyrrole, polyvinylimidazole, thiol-functionalized polymers, carbonyl-functionalized polymers, or piperidine-functionalized polymers;
(ii) the sulfonic acid group-containing polymer layers comprising at least one of sulfonated polyvinylpyridine, sulfonated polypyrrole, sulfonated polybenzimidazole, sulfonated polybenzothiazole, polysulfobetaine, perfluorosulfonic acid polymers, sodium polystyrene sulfonate, or sodium polyethylene sulfonate; and/or

(c) the third polymer layer is selected from at least one of cationic polymer layers, anionic polymer layers or amphoteric polymer layers,

wherein (i) the cationic polymer layers comprising at least one of polyethyleneimine, polyallylamine, polyacrylamide, polyaniline, poly(2-(dimethylamino)ethyl methacrylate), chitosan, polylysine, or perfluorosulfonic acid polymers;
(ii) the anionic polymer layers comprising at least one of polyacrylic acid, sodium polystyrene sulfonate, sodium polyethylene sulfonate, cellulose acetate, sodium alginate, hyaluronic acid, heparin, or polyphosphoric acid;
(iii) the amphoteric polymer layers comprising polybetaine.

**6.** The sensor protective film of claim 1, wherein a thickness of the sensor protective film is in the range from 5 μm to 50 μm.

**7.** The sensor protective film of claim 1, wherein: a water absorption rate of less than or equal to 20%; and the sensor protective film exhibits a swelling ratio of less than or equal to 8%.

**8.** A method for preparing the sensor protective film, wherein the method comprising:

(a) solidifying a first polymer film solution to form the first polymer layer, wherein the first polymer film solution comprises a nitrogen-containing cationic polymer, a phosphorus-containing cationic polymer, or a combination thereof;
(b) applying at least one of a second polymer film solution or a third polymer film solution onto a surface of the first polymer layer,

wherein (i) the second polymer film solution comprising a sulfonic acid group-containing polymer, a neutral ligand-containing polymer capable of coordinating with silver ions, or a combination thereof;
(ii) the third polymer film solution comprising at least one of an anionic group-containing polymer or a cationic group-containing polymer; and

(c) curing the applied solution(s) to form at least one of the second polymer layer or the third polymer layer, thereby obtaining the sensor protective film.

**9.** The method of claim 8, wherein:

(a) the nitrogen-containing cationic polymer, the phosphorus-containing cationic polymer, or a combination thereof is present in the first polymer film solution in an amount of 5 wt% to 50 wt%, based on the total weight of the first polymer film solution; and/or
(b) the sulfonic acid group-containing polymer, the silver-ion-coordinating neutral ligand-containing polymer, or a combination thereof is present in the second polymer film solution in an amount of 5 wt% to 50 wt%, based on the total weight of the second polymer film solution; and/or
(c) the anionic group-containing polymer and/or the cationic group-containing polymer is present in the third polymer film solution in an amount of 5 wt% to 50 wt%, based on the total weight of the third polymer film solution.

**10.** The method of claim 9, wherein:

(a) the first polymer film solution further comprises an epoxy-based crosslinking agent at a mass ratio of 1:100 to

3:20 relative to the nitrogen and/or phosphorus cationic group polymer; and/or
(b) the second polymer film solution further comprises an epoxy-based crosslinking agent at a mass ratio of 1:100 to 3:20 relative to the sulfonic acid group and /or neutral ligand structure polymer.

**11.** The method of claim 10, wherein:

(a) the nitrogen-containing cationic polymer, the phosphorus-containing cationic polymer, or a combination thereof is selected from at least one of quaternized polyvinylpyridine, quaternized polypyrrole, quaternized polybenzimidazole, quaternized polybenzothiazole, or polyquaternized amphoteric ionic polymer; and/or
(b) the epoxy-based crosslinking agent in each of the first polymer film solution and the second polymer film solution is independently selected from at least one of polyethylene glycol diglycidyl ether, poly(propylene glycol) diglycidyl ether, poly(dimethylsiloxane) diglycidyl ether, bisphenol A diglycidyl ether, 1,4-butanediol diglycidyl ether, tri(4-hydroxyphenyl) methane triglycidyl ether, or trimethylolpropane triglycidyl ether; and/or
(c) the sulfonic acid group-containing polymer, the silver-ion-coordinating neutral ligand-containing polymer, or a combination thereof is selected from at least one of polysulfobetaine, perfluorosulfonic acid polymer, sodium polystyrene sulfonate, sodium polyethylene sulfonate, polyvinylpyridine, polypyrrole, polyvinylimidazole, thiol polymer, carbonyl polymer, piperidine polymer, sulfonated polyvinylpyridine, sulfonated polypyrrole, sulfonated polybenzimidazole, or sulfonated polybenzothiazole;and/or
(d) the anionic group-containing polymer and/or the cationic group-containing polymer is selected from at least one of polyallylamine, polyethyleneimine, polyacrylamide, polyaniline, poly(2-(dimethylamino)ethyl methacrylate), polyacrylic acid, sodium polystyrene sulfonate, sodium polyethylene sulfonate, chitosan, cellulose acetate, sodium alginate, hyaluronic acid, heparin, polylysine, perfluorosulfonic acid polymer, polyphosphoric acid, or polybetaine.

**12.** An electrochemical sensor, wherein the electrochemical sensor comprising a sensor substrate and a sensor protective film disposed on the sensor substrate surface, wherein the sensor protective film is as defined in any one of claims 1 to 7.

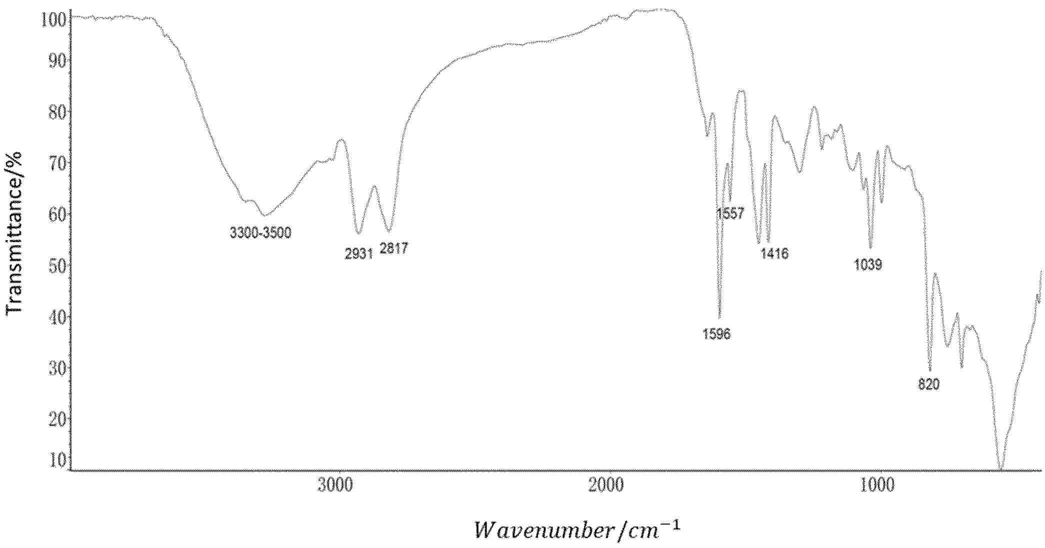
Transmittance/%
100
90
80
70
60
50
40
30
20
10
3300-3500
2931
2817
1596
1557
1416
1039
820
3000
2000
1000
$Wavenumber/cm^{-1}$

FIG. 1

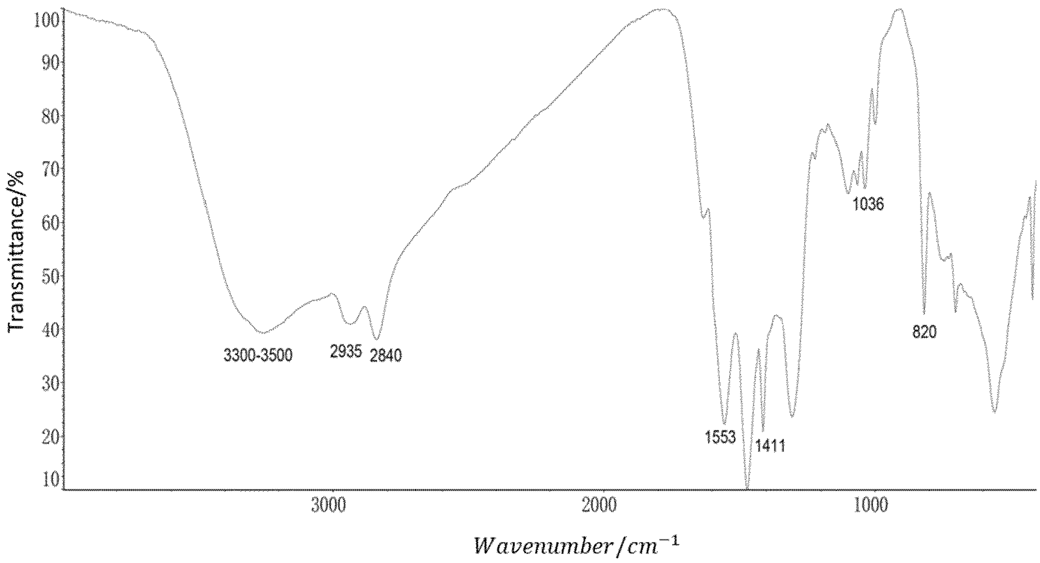
Transmittance/%
100
90
80
70
60
50
40
30
20
10
3300-3500
2935
2840
1553
1411
1036
820
3000
2000
1000
$Wavenumber/cm^{-1}$

FIG. 2

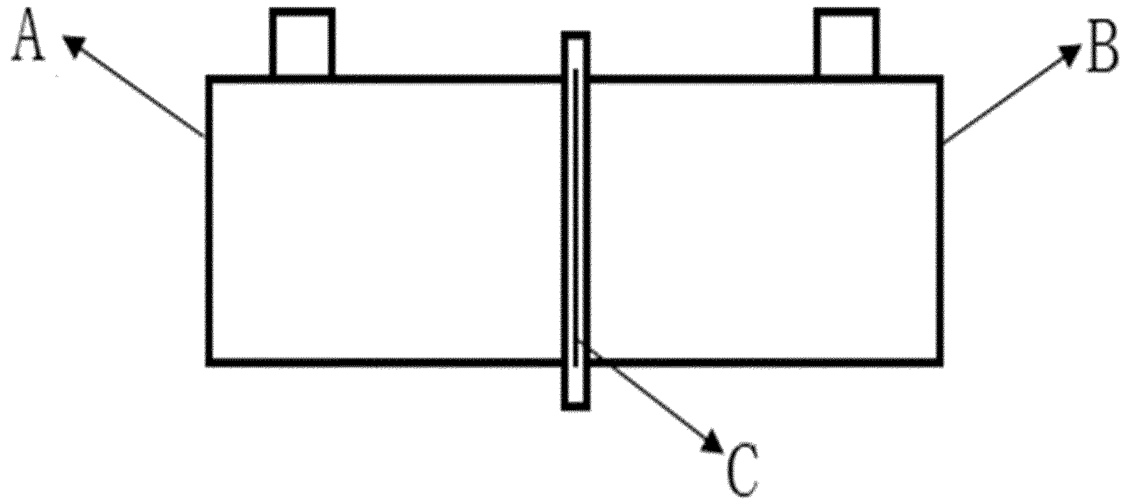


FIG. 3

Europäisches Patentamt
European Patent Office
Office européen des brevets

# EUROPEAN SEARCH REPORT

Application Number
EP 25 17 9660

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2010 0122874 A (IAC IN NAT UNIV CHUNGNAM [KR]) 23 November 2010 (2010-11-23) | 1,4 | INV. A61B5/00 B32B27/08 |
| Y | * paragraphs 8-10,15,16 and 19-23 of the | 6,7,12 | |
| A | translated document * | 2,3,5, 8-11 | |
| X | KR 2010 0112662 A (IAC IN NAT UNIV CHUNGNAM [KR]) 20 October 2010 (2010-10-20) | 1,4 | |
| Y | * paragraphs 1-6, claims 1-5 of the translated document * | 6,7,12 | |
| A | CN 1 281 323 C (POREX CORP [US]) 25 October 2006 (2006-10-25) * the whole document * | 1-12 | |

TECHNICAL FIELDS SEARCHED (IPC)

A61B
B32B

The present search report has been drawn up for all claims

1

| Place of search | Date of completion of the search | Examiner |
|---|---|---|
| Munich | 22 October 2025 | Michalitsch, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 17 9660

This annex lists the patent family members relating to the patent documents cited in the above-mentioned European search report. The members are as contained in the European Patent Office EDP file on
The European Patent Office is in no way liable for these particulars which are merely given for the purpose of information.

22-10-2025

| Patent document cited in search report | Publication date | Patent family member(s) | Publication date |
|---|---|---|---|
| KR 20100122874 A | 23-11-2010 | NONE | |
| KR 20100112662 A | 20-10-2010 | NONE | |
| CN 1281323 C | 25-10-2006 | AT E353707 T1 | 15-03-2007 |
| | | CN 1578702 A | 09-02-2005 |
| | | DE 60218187 T2 | 31-10-2007 |
| | | EP 1427532 A1 | 16-06-2004 |
| | | JP 4652684 B2 | 16-03-2011 |
| | | JP 2005501758 A | 20-01-2005 |
| | | US 2003096424 A1 | 22-05-2003 |
| | | US 2003124332 A1 | 03-07-2003 |
| | | US 2006280931 A1 | 14-12-2006 |
| | | WO 03020425 A1 | 13-03-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202410704426
- GB 130221991 A
- GB 92861998 A


### Non-patent literature cited in the description


- *CHEMICAL ABSTRACTS*, 9017-40-7
- *CHEMICAL ABSTRACTS*, 74-96-4
- *CHEMICAL ABSTRACTS*, 1633-83-6
- *CHEMICAL ABSTRACTS*, 9002-98-6
- *CHEMICAL ABSTRACTS*, 39443-66-8
- *CHEMICAL ABSTRACTS*, 36366-26-4
- *CHEMICAL ABSTRACTS*, 25704 - 18 - 1
- *CHEMICAL ABSTRACTS*, 25928-81-8
- *CHEMICAL ABSTRACTS*, 74-88-4
- *CHEMICAL ABSTRACTS*, 3637 - 26 - 1
- *CHEMICAL ABSTRACTS*, 9003-01-4